(19) Europäisches Patentamt
European Patent Office
Office européen des brevets

(11) Publication number: **0 552 386 A1**

(12) **EUROPEAN PATENT APPLICATION**

(21) Application number: **92100432.1**

(22) Date of filing: **13.01.92**

(51) Int. Cl.5: **C07D 307/79**, C07D 333/54, C07D 295/135, A61K 31/38, A61K 31/40, A61K 31/34

(43) Date of publication of application: **28.07.93 Bulletin 93/30**

(84) Designated Contracting States: **AT BE CH DE DK ES FR GB IT LI LU NL SE**

(71) Applicant: **WARNER-LAMBERT COMPANY**
**201 Tabor Road**
**Morris Plains New Jersey 07950(US)**

(72) Inventor: **Horwell, David Christopher**
**8, West Hill**
**Foxton, Campridge(GB)**
Inventor: **Rees, David Charles**
**33, Norwich Street**
**Cambridge CB2 2JB(GB)**
Inventor: **Goel, Om Prakash**
**3995, Holden Drive**
**Ann Arbor, Michigan 48103(US)**

(74) Representative: **Mansmann, Ivo et al**
**c/o Gödecke AG - Patentabteilung Postfach**
**569 Mooswaldallee 1-9**
**W-7800 Freiburg (DE)**

(54) **2-Amino-3 or 6-methoxycyclohexyl amide derivatives.**

(57) The invention concerns novel 2-amino-3- or 6-methoxycyclohexyl amides of the formula

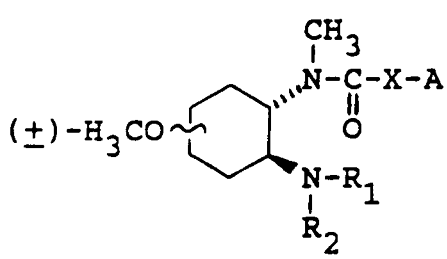

I

having analgesic and neuroprotective activity. The compounds bind selectively to the kappa opioid receptor. Pharmaceutical compositions containing the compounds, methods of using them, and processes for preparing them are also disclosed.

EP 0 552 386 A1

## CROSS REFERENCE

This invention relates to EP-A-0 372 466. The instant invention covers compounds not disclosed in the prior case. Compounds substituted on the cyclohexyl moiety by a methoxy group at the 3 or at the 6 position are the instant invention while the compounds of EP-A-0 372 466 are substituted in the 4- or 5-position. The compounds with the 3- or 6-methoxy substituent exhibit an unexpected and particularly high selectivity for the kappa versus mu opioid receptor in the in vitro receptor binding assay.

## BACKGROUND OF THE INVENTION

The search for strong analgesics which also possess minimal potential for dependency has been among the highest priority efforts in pharmaceutical research. These research efforts have, to a great extent, involved chemical modification of the opiate structure and the discovery of novel compounds which possess morphine-like activity.

The concept of multiple opioid receptors has been supported by studies with nalorphine and a series of benzomorphans which display unusual pharmacological properties dissimilar from morphine, yet blocked by the selective opioid antagonists. [See, for example, W. R. Martin, et al, J. Pharmacol. Exp. Ther., **197**: 517-532 (1976).]

The existence of multiple types of opioid receptors is of importance because it suggests the possibility of separating the desirable analgesic and psychotherapeutic effects of a drug compound from the undesirable abuse potential or habituating effects. United States Patent 4,098,904 discloses certain cis- and trans-N-(2-aminocycloaliphatic) benzamide compounds having analgesic activity.

United States Patent 4,145,435 describes certain 2-amino-cycloaliphatic amide compounds as analgesics. In particular, trans-3,4-dichloro-N-[2-(1-pyrrolidinyl)-cyclohexyl]benzacetamide is reported to possess selective kappa agonist activity, and therefore, to possess analgesic activity without attendant dependence liability. [See P. V. Vonvoigtlander, et al, J. Pharmacol. Exp. Ther., **224**: 7-12 (1983).]

United States Patent 4,212,878 discloses certain N-[(4-mono- or di-oxygen-group-substituted-1-amino-cyclohex-1-yl)methyl)phenylacetamides, particularly 2-(3,4-dichlorophenyl)-N-[[8-(1-pyrrolidinyl)-1,4-dioxaspiro[4.5]dec-8-yl]methyl]acetamide having analgesic properties.

United States Patent 4,359,476 and its continuation-in-part 4,460,600 disclose certain N-[2-amino(oxy or thio group) substituted cyclcaliphatic]benzeneacetamide and -benzamide compounds having the oxy- or thio group substituents on a cycloaliphatic ring carbon adjacent to either of the nitrogen-bearing carbon atoms of the cycloaliphatic ring. These compounds, having analgesic activity are typified by 4-bromo-N-[3-methoxy-2-(1-pyrrolidinylcyclohexyl]-N-methylbenzamide.

United States Patent 4,598,087 and its divisional, United States Patent 4,677,122, disclose certain oxy- or thioacetamides of trans-1,2-diamino-cyclohexane having analgesic activity. These compounds are typified by trans-2-(2,3-dichlorophenoxy)-N-methyl-N-[2-(1-pyrrolidinyl)cyclohexyl]-acetamide.

United States Patent 4,656,182 discloses certain trans-N-methyl-N-[2-(1-pyrrolidinyl)cyclohexyl]benzo-[b]thiophene acetamides having analgesic activity.

United States Patent 4,663,343 discloses certain trans-N-methyl-N-[2-(1-pyrrolidinyl)cyclohexyl]-naphthalenyloxy- and naphthalenylthioacetamides having analgesic activity.

United States Patent 4,737,493 discloses certain substituted phenoxy-, 1-, and 2-naphthalenyloxy, indenyl-, indolyl-, benzofuranyl-, and benzo[b]thiofuranylcarboxamides of 7,8-(substituted-diamino)-1-oxaspiro[4.5]decanes useful as analgesic agents.

United States Patent 4,463,013 discloses certain oxygen substituted amino-cyclohexyl-benzeneacetamides as diuretics.

United States Patent 4,438,130 discloses certain mono-axa-, thiaspiro-cyclic-benzeneacetamide and benzamide compounds useful as analgesics.

United States Patents 4,906,655 and 5,019,588 disclose certain 1,2-cyclohexylaminoaryl amides useful as analgesic agents.

## SUMMARY OF THE INVENTION

The invention relates to a novel series of monomethoxycyclohexyl amides which possess selective kappa receptor analgesic activity. The compounds show a surprising increase in activity in the nociceptive assay of the rat paw pressure test of M. B. Tyers, Brit. J. Pharmacol., 69:503-512 (1980) when compared with compounds known in the art.

The invention covers novel 2-amino-3 or 6-methoxycyclohexyl amide derivatives of the formula

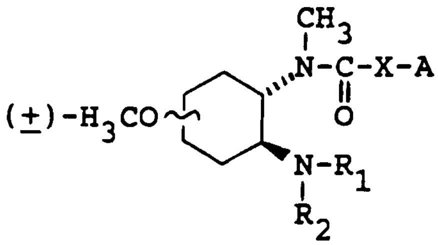

I

wherein the (±)-$H_3CO$~ group is in the 3- or 6- position and the two nitrogen atoms attached to the cyclohexane moiety are trans to one another;
X is $CH_2$ or direct bond,
$R_1$ is methyl,
$R_2$ is selected from the group consisting of:
hydrogen,
alkyl of from one to six carbon atoms,

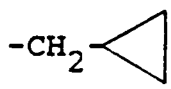

,

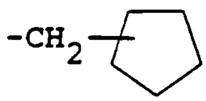

,

$-CH_2CH=CR_3R_4$,
$-CH_2C=R_3$,
-2- or 3-thienyl, or

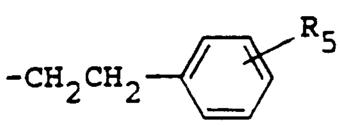

wherein $R_3$ and $R_4$ are each independently hydrogen or methyl,
$R_5$ is selected from the group consisting of:
hydrogen,
fluorine,
chlorine,
bromine,
lower alkyl, and
lower alkoxy;
or $R_1$ and $R_2$ may be taken together with the nitrogen atom to which they are attached to from a ring

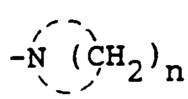

wherein n is an integer of from 3 to 8;
A is selected from the group consisting of:

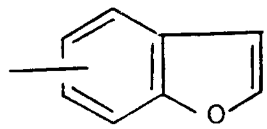 ,

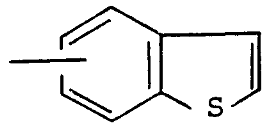 ,

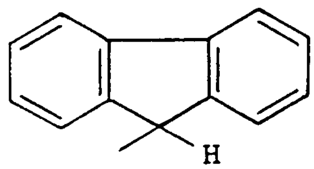 ,

phenyl,
phenyl substituted with from one to four halogen atoms,
phenyl substituted with one or two alkyl groups of from one to four carbon atoms,
phenyl substituted with one or two alkoxy groups of from one to four carbon atoms, or
phenyl substituted with one or two alkyl groups of from one to four carbon atoms and one or two halogen atoms;
or a pharmaceutically acceptable acid salt thereof and especially (1α,2α,6β)-N-[2-methoxy-6-(1-pyrrolidinyl)-cyclohexyl]-N-methyl-4-benzofuranacetamide and (1α,2β, 3α)-N-[3-methoxy-2-(1-pyrrolidinyl)-cyclohexyl]-N-methyl-4-benzofuranacetamide and the pharmaceutically acceptable acid addition salts especially the monohydrochlorides.

The invention also includes pharmaceutical compositions comprising an analgesically effective amount of the above compounds in admixture with a pharmaceutically acceptable carrier or excipient and the use of the above compounds for preparing a pharmaceutical composition for treating pain in a patient suffering therefrom.

The invention further includes pharmaceutical compositions comprised of a neuroprotective amount of the above compounds in admixture with a pharmaceutically acceptable carrier or excipient as well as the use of the above compounds for preparing a pharmaceutical composition for treating stroke and/or cerebral ischemia in a patient suffering therefrom.

The invention further includes methods for preparing the compounds recited above and a process for preparing the above pharmaceutical compositions.

A method for preparing compounds of the invention wherein a substituent is

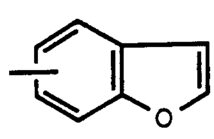 or 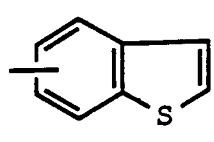

and the methoxy moiety is attached at 3- or 6- position comprises epoxidizing 3-methoxycyclohexene to produce a mixture of the trans and cis epoxides in a ratio of 2:1 and separating them by fractional distillation.

The trans epoxide is reacted with pyrrolidine to produce a mixture of two isomeric amino alcohols. The mixture is treated with methanesulfonyl chloride and then N-methylbenzylamine to produce N-benzyldiamine which is converted to the salt and catalytically hydrogenated. The diamine is then reacted with a benzofuran or benzothiophene acetic acid to produce a compound of the invention.

The cis epoxide undergoes the same reaction sequence.

Alternatively the trans epoxide is first reacted with N-benzylmethylamine to produce an amino alcohol which is converted to the salt; the mixture crystal resulting is mechanically separated, the largest crystal is catalytically debenzylated and the product reacted with 4-benzofuranacetyl chloride to produce a compound

of the invention.

The compounds of the present invention include solvates, hydrates, and pharmaceutically acceptable acid addition salts of the basic compounds of formula I above.

By virtue of the basic nitrogen on the cyclohexane moiety, pharmaceutically acceptable salts of compounds of the present invention may be prepared by reaction with appropriate acids. Suitable acids for the formation of pharmaceutically acceptable salts of the compounds of this invention form a class well known to practitioners of the pharmaceutical formulation arts (cf. S. M. Berge, et al, "Pharmaceutical Salts" in J. Pharm. Sci., 66: 1-19 (1977)), and include such acids as hydrochloric, hydrobromic, hydriodic, sulfuric, nitric, phosphoric, acetic, benzoic, citric, maleic, tartaric, succinic, gluconic, ascorbic, sulphamic, oxalic, pamoic, methanesulfonic, benzenesulfonic, ethanesulfonic, hydroxyethanesulfonic, and related acids and mixtures thereof.

The salts are generally prepared by reacting the free base with one equivalent of the desired acid in an appropriate unreactive solvent, followed by collection of the salt by filtration or recovery upon removal of the solvent. The free base may be regenerated, if desired, by reaction of the salt with one equivalent of a base such as sodium hydroxide, sodium bicarbonate, sodium carbonate, and the like. The salts may differ from the free base form of compounds of this invention in properties such as melting point and solubility in polar solvents, but are otherwise considered equivalent for the purposes of this invention.

The compounds of the present invention contain three or more asymmetric carbon atoms. The compounds exist in various stereo- and regio-isomeric forms and mixtures thereof. The present invention contemplates all stereo- and regio-isomeric forms of the compounds of formula I above. Both the (+) and (-) and the (±) are contemplated by the invention.

The individual stereo compounds are obtained, if desired, from a mixture of different forms by known methods of resolution such as the formation of diastereomers followed by recrystallization.

The compounds of the present invention possess significant analgesic activity with the potential for minimum dependence liability due to their selective kappa opioid receptor binding properties. In addition to producing analgesia, compounds which are selective kappa antagonists, such as the compounds of this invention, also cause opioid receptor-mediated sedation, diuresis, and corticosteroid elevation. Accordingly, the compounds of this invention may also be useful as diuretics and psychotherapeutic agents as well as analgesics.

The compounds of the present invention also have application in congestive heart failure, advanced hepatic cirrhosis, nephrotic syndrome, chronic renal failure, trauma associated with surgery, emotional and physical stress, endocrine disorders, syndrome of inappropriate antidiuretic hormone secretion and therapy with certain pharmacologic drug agents such as certain sulphonyl ureas, certain biguanides such as phenformin and metformin, clofibrate, certain tricycles such as carbamazepine, amitriptyline, thiothixene, fluphenazine and thioridazine, certain antineoplastic agents, certain analgesics and certain natriuretic diuretics.

The compounds of the present invention also have neuroprotective indications. As such they are useful in the treatment of stroke and the treatment of cerebral ischemia (P. F. Von Voightlander in Brain Research **435**: 174-180 (1987)) and A. H. Tang, et al. in Brain Research **403**: 52-57 (1987)).

Representative compounds of the present invention demonstrate positive activity in standard laboratory analgesic tests in animals such as mice. The $MPE_{50}$ doses for several representative compounds of this invention in the standard rat paw pressure analgesia test M. B. Tyers, Brit. J. Pharmacol., (1980), 69: 503-512 are presented in Table I below.

Compound 17 in the table exhibits a particularly high selectivity for the kappa versus mu opioid receptor in the in vitro binding assay.

TABLE I

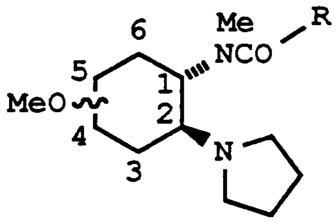

$R^1$ =

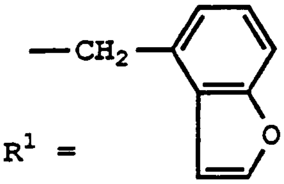

$R^2$ =

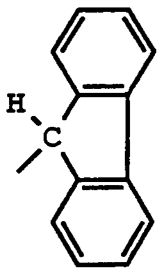

| Compound Number | Structure (Position of OMe) | R | Opioid Binding ($K_i$nM) | | | Rat Paw Pressure $MPE_{50}$ IV | $MPE_{50}$ mg/kg PO | PO/IV Ratio |
|---|---|---|---|---|---|---|---|---|
| | | | kappa | mu | mu/k | | | |
| 10 | 4-ß | $R^1$ | 7.1 ± 4 | 3300 ± 1900 | 465 | 0.07 | | |
| 5 | 4-α | $R^1$ | 1.72 | 210 | 122 | 1.38 | 18.4 | 13 |
| 6 | 5-ß | $R^1$ | 4.13 | 2020 | 490 | 1.89 | 20.2 | 10.6 |
| 7 (Sch 1) | 5-α | $R^1$ | 24 ± 4 | 4300 ± 400 | 179 | 1.5 | | |
| 8 | 4-α | $R^2$ | 0.84 ± 0.9 | 28 ± 1.3 | 33 | — | | |
| 9 | 5-ß | $R^2$ | 6.7 ± 1.3 | 2130 ± 176 | 318 | >1 | | |
| 7 (Sch 2) | 3-ß | $R^1$ | 7.36 | 2220 | 302 | | | |
| 12 (Sch 3) | 3-α | $R^1$ | 253 | 359 | 1.4 | — | — | — |
| 17 (Sch 4) | 6-α | $R^2$ | 3.45 | 5100 | 1480 | 2.5 | | |

$K_i$ values represent the mean ± (standard error of the mean) from concentration-response curves performed in triplicate from each of the least two separate experiments.

$MPE_{50}$ values represent the dose required to produce 50% of the maximum possible analgesic effect. They are derived from a single experiment with six animals per dose level.

Representative compounds of the present invention were also tested in vitro to determine the extent of opioid receptor binding, and were found to bind selectively to the kappa opioid receptor site with evidence of little or no binding to the mu or delta opioid receptors.

Measurement of the kappa opioid receptor binding activity of compounds of the present invention was made by the following method. Guinea pig brain homogenates were prepared fresh daily utilizing the method of Gillan et al, Br. J. Pharmacol. (1980) 70: 481-490.

The binding of tritiated etorphine to brain homogenates was measured in the presence of unlabeled competitor compounds of the present invention with 200 nanomolar D-alanine-D-leucine-enkephalin (acronym DADLE) and 200 nanomolar D-ala-MePheGly-ol-enkephalin (acronym DAGO) added to saturate the delta and mu opioid receptors, respectively. The reaction was terminated by rapid filtration and the radioactivity bound to the filters counted by liquid scintillation spectrophotometry.

Measurement of the mu and delta opioid receptor binding activity of the compounds of this invention was made by the following method. Guinea pig brain homogenates were freshly prepared daily by the method of Gillan, et al, cited above.

Homogenates were incubated for 150 minutes at 0°C with either tritiated DAGO to measure mu receptor binding activity, or with tritiated DADLE in the presence of a ten-fold excess of unlabeled DAGO to measure delta opioid receptor binding. Nonspecific binding was determined in the presence of $10^{-6}$ molar

DAGO and $10^{-6}$ molar DADLE.

Reactions were terminated by rapid filtration and the radioactivity bound to the filters counted by liquid scintillation spectrophotometry.

The data were analyzed by the methods of Scatchard, Ann. N.Y. Acad. Sci., 51: 660-672 (1949) and Hill, J. Physiol., 40: IV-VIII (1910). The inhibition of the binding of tritiated etorphine, DAGO and DADLE by cold ligands was determined from the regression of log percentage inhibition of specific binding or log concentration of cold ligand. The inhibition constant, $K_i$, was calculated from the equation:

$$K_i = \frac{IC_{50}}{1 + [L]/K_D}$$

where [L] is the concentration of the labeled ligand and $K_D$ is the equilibrium dissociation constant.

Schemes 2-4 below illustrate chemical syntheses of some of the compounds of the present invention. All compounds are racemic mixtures. They are prepared by epoxidizing commercially available 3-methoxycyclohexene (also named 2-cyclohexene-1-ol, methyl ether) by the action of m-chloroperbenzoic acid in dichloromethane to yield a mixture of trans and cis 3-methoxycyclohexane epoxides 1 and 2 in a ratio of 2:1. The trans and cis epoxides were carefully separated by fractional distillation to greater than 95% isomeric purity. In Synthetic Scheme 2, the trans epoxide 1, is reacted with pyrrolidine to give a mixture of two isomeric amino alcohols 3 and 3a. This mixture of amino alcohols is treated with methanesulfonyl chloride and subsequently with N-methylbenzylamine to yield N-benzyldiamine 4. The compound 4 is converted to the hydrochloride salt 5 and catalytically hydrogenated to afford 97% pure 6. The diamine 6 was reacted with benzofuran acetic acid in presence of carbonyl-diimidazole to afford Compound 7 of this invention.

In Scheme 3 the cis epoxide 2 was reacted in the same sequence as above which afforded the Compound 12 of shown stereochemistry.

In Scheme 4, the trans epoxide 1 is first reacted with N-benzylmethylamine to give amino alcohol 13. This amino alcohol is treated with methanesulfonyl chloride and subsequently with pyrrolidine to yield 14 as a mixture of four components as determined by GC in a ratio of 1:4:7:4. When 13 was converted to the hydrochloride salt, a mixture of crystals was formed from which the largest brown color crystals were mechanically separated and found to be a single isomer 15 based on spectroscopic evidence. On catalytic debenzylation with hydrogen 16 was formed. This was reacted with 4-benzofuranacetyl chloride to yield 17.

SCHEME 2

(all compounds are racemic)

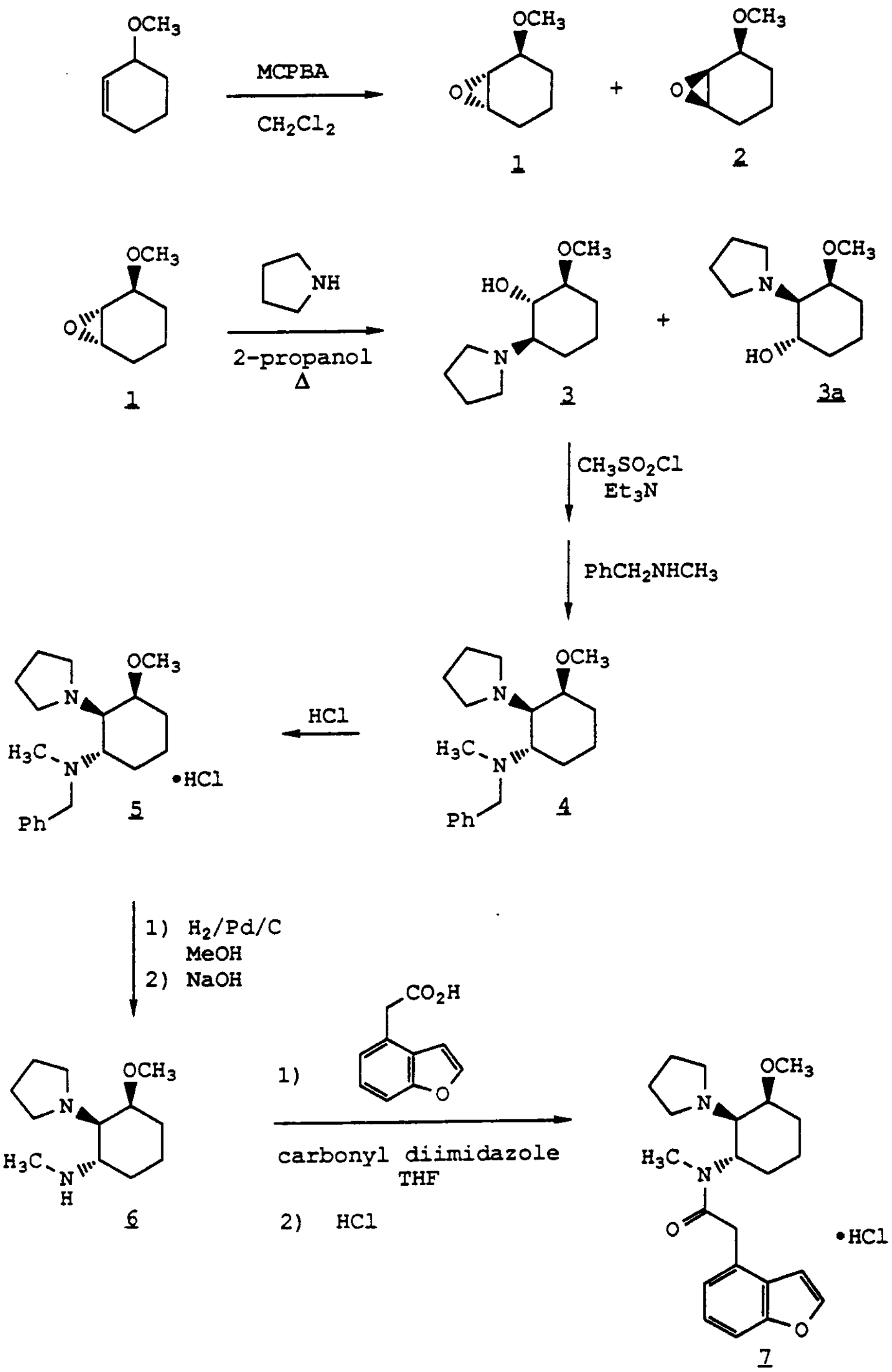

SCHEME 3

(all compounds are racemic)

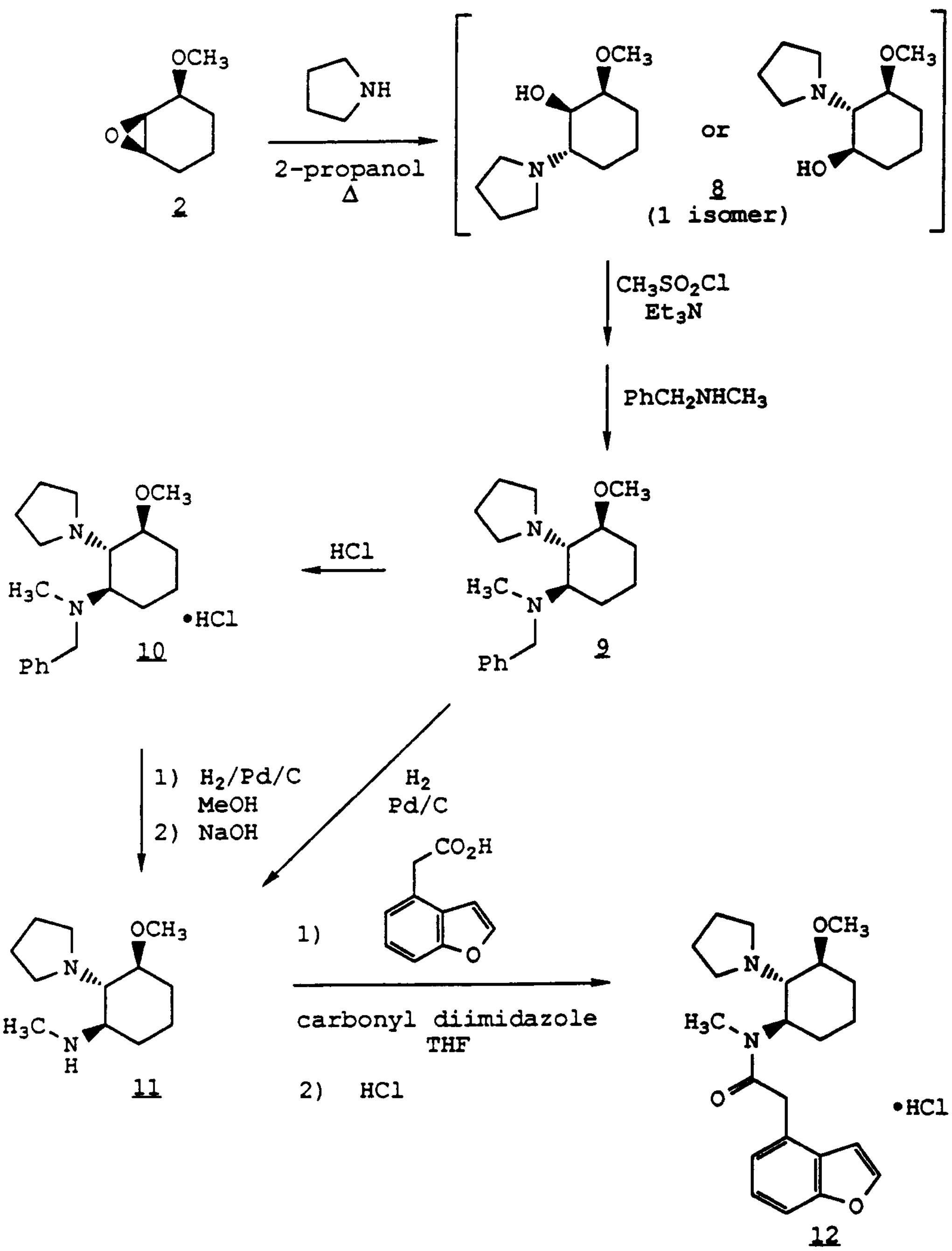

SCHEME 4

(all compounds are racemic)

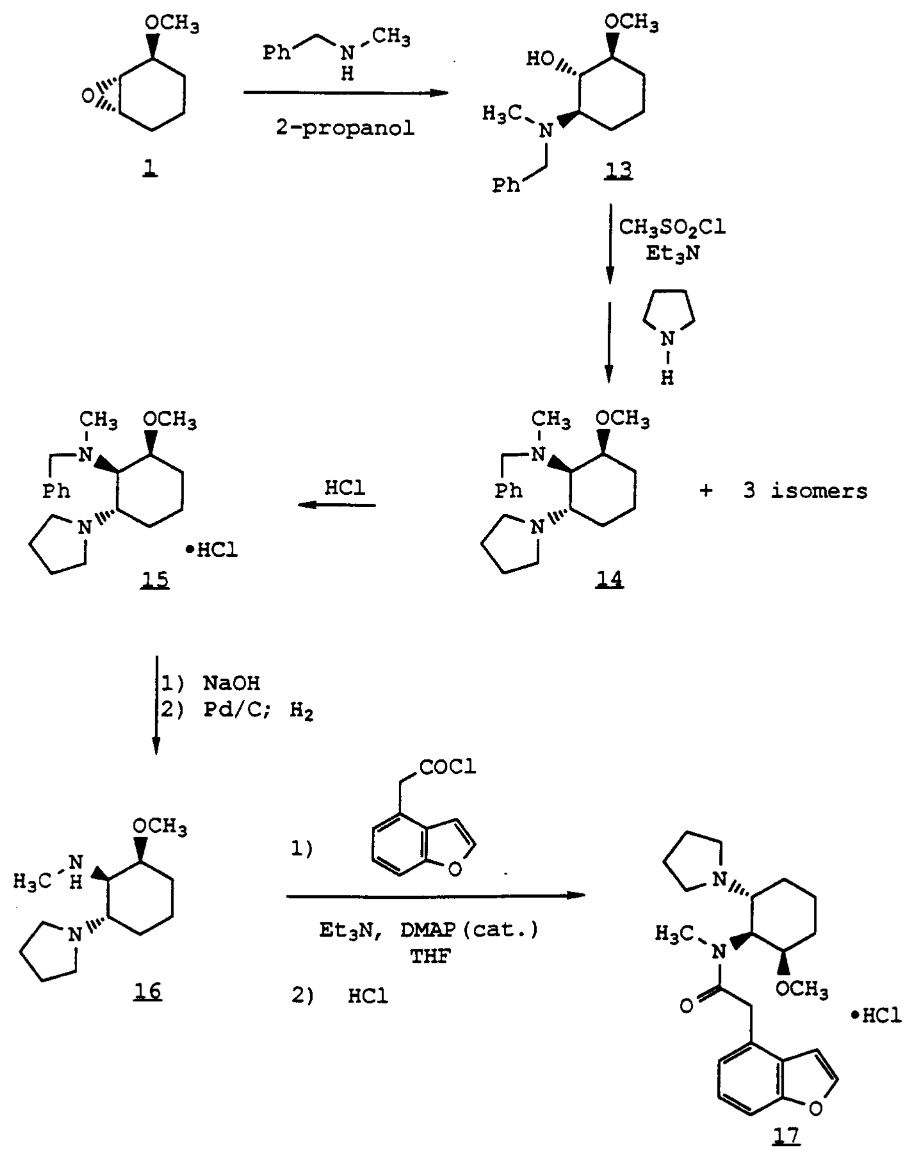


1, 13, 14, 15, 16, 17

Compounds of the present invention and/or their nontoxic, pharmaceutically acceptable salts may be administered to mammals orally in combination with conventional compatible carriers in solid or in liquid form. These oral pharmaceutical compositions may contain conventional ingredients such as binding agents selected from syrups, acacia, gelatin, sorbitol, tragacanth, polyvinylpyrrolidone, and mixtures thereof. The compositions may further include fillers such as lactose, mannitols, starch, calcium phosphate, sorbitol, methylcellulose, and mixtures thereof.

These oral compositions may also contain lubricants such as magnesium stearate, high molecular weight polymers such as polyethylene glycol, high molecular weight fatty acids such as stearic acid, silica, or agents to facilitate disintegration of the solid formulation such as starch, and wetting agents such as sodium lauryl sulfate.

The solid oral compositions may take any convenient form such as tablets, lozenges, capsules, or dry powders which may be reconstituted with water or other suitable liquid prior to administration.

Liquid form pharmaceutical compositions may take the form of solutions, suspensions, or emulsions. The liquid forms may contain flavorants, sweeteners, and/or preservatives such as alkyl p-hydroxybenzoates. They may further contain suspending agents such as sorbitol, glucose, or other sugar syrups, methyl-, hydroxymethyl-or carboxymethylcellulose, and gelatin, emulsifying agents such as lecithin or sorbitol monooleate, and conventional thickening agents.

Liquid compositions may optionally be encapsulated in, for example, gelatin capsules in an effective amount.

The compounds of the invention may also be administered to mammals rectally in the form of suppositories. For preparing suppositories, a low-melting wax such as a mixture of fatty acid glycerides or cocoa butter is first melted, and the active ingredient is dispersed homogeneously in the melt. The mixture is then poured into convenient sized molds and allowed to cool and solidify.

Preferably, the pharmaceutical compositions of this invention are in unit dosage form. In such form, the preparation is subdivided into unit doses containing appropriate amounts of the active component. The unit doses can be a packaged preparation with the package containing discrete quantities of the preparation. For example, the package may take the form of packaged tablets, capsules, and powders in envelopes, vials, or ampoules. The unit dosage form can also be a capsule, cachet, or tablet itself or can be the appropriate number of any of these in package form.

The quantity of active compound in a unit dose of preparation may be varied or adjusted from about 0.5 mg to about 350 mg according to the particular application and the potency of the active ingredient.

When employed systematically in therapeutic use as analgesic agents in the pharmaceutical method of this invention, the compounds are administered at doses of from about 0.05 mg to about 2.0 mg of active compound per kilogram of the recipient.

The following examples are provided to enable one skilled in the art to practice the present invention. These examples are not intended in any way to limit the scope of the invention but are intended as illustrative thereof.

EXAMPLE 1

4-Methoxycyclohexene epoxide (1)

m-Chloroperoxybenzoic acid (3.5 g, 20 mmol) in 1:1 dichloromethane-carbon tetrachloride (70 ml) was added over 25 minutes to a stirred solution of 4-methoxycyclohexene (2.0 g, 18 mmol) in carbon tetrachloride (10 ml) at -5 °C. After 5 hours the mixture was allowed to warm to room temperature and after 2 hours the slurry was filtered. The filtrate was washed with 5% aqueous sodium bisulphite (40 ml) then saturated aqueous sodium carbonate (2 x 40 ml). The resulting solution was dried ($K_2CO_3$) and distilled using a vigreaux column at atmospheric pressure to give the epoxides (1) as a mixture of two diastereoisomers (1.0 g, 7.8 mmol), 43%), bp 175-176 °C (760 mm Hg); i.r. (neat) 2938, 1104 $cm^{-1}$; $\delta$ ($CDCl_3$, 300 MHz) 3.22 (1H,m); 3.24 (s) and 3.22 (s) together (3H); 3.05 (2H,m), 2.3-1.2 (6H,m); m/e ($EI^+$) 129 (11%), 111 (35%), 97 (65%), 58 (100%); Anal. $C_7H_{12}O_2$ requires C, 65.60; H, 9.44. Found C, 65.57; H, 9.60%.

EXAMPLE 2

Amino alcohols (2)

The epoxides (1) (2.0 g, 15.6 mmol) and N-benzylmethylamine (3.0 g, 25 mmol) were dissolved in propan-2-ol (10 ml) and heated under reflux for 20 hours. The resulting solution was distilled to give the amino alcohols (2) as a mixture of three isomers (3.2 g, 13 mmol, 83%), bp 133-134 °C/ 0.05 mbar; i.r. (neat) 3460, 2939, 2866 $cm^{-1}$; $\delta$ ($CDCl_3$, $D_2O$) 7.3 (5H,m); 4.0-2.7 (7H,m, strong s at 3.36, 3.30, 3.29); 2.4-1.2 (10H,m, strong s at 2.19, 2.18, 2.15); m/e ($EI^+$) 249 (10%), 218 (8%), 190 (100%); Anal. $C_{15}H_{23}NO_2$ requires C, 72.25; H, 9.30; N, 5.62. Found C, 72.13; H, 9.22; N, 5.56%. $^1H$ nmr spectra of the individual isomers were obtained after separation by silica gel chromatography using 20:1 dichloromethane-methanol. $\delta$ ($CDCl_3$, $D_2O$) Isomer A: 7.24 (5H,m), 3.61 (1H,d,J = 14); 3.45 (1H,J = 14); 3.43 (1H,m); 3.36 (3H,s); 3.18 (1H,m); 2.36 (1H,m); 2.19 (3H,s); 2.27-1.99 (3H,m); 1.22 (3H,m). Isomer B: 7.28 (5H,m); 3.70 (1H,d,J = 15 Hz); 3.65 (1H,m); 3.45 (1H,m); 3.44 (1H,d,J = 15 Hz); 3.30 (3H,s); 2.83 (1H,m); 2.15 (3H,s); 2.20-1.85 (3H,m); 1.60 (1H,m); 1.30 (2H,m). Isomer C: 7.28 (5H,m); 3.79 (1H,m); 3.74 (1H,d,J = 15 Hz); 3.55 (1H,m); 3.45 (1H,d,J = 15 Hz); 3.29 (3H,s); 2.42 (2H,m); 2.18(3H,s); 2.05 (1H,m); 1.55 (2H,m); 1.30 (2H,m).

EXAMPLE 3

N-benzyl diamines (3)

The diastereoisomeric mixture of amino alcohols (2) (8.0 g, 32 mmol) was dissolved in dichloromethane (105 ml) and triethylamine (7.1 ml), cooled to -10 °C, and treated with methenesulphonyl chloride (2.7 ml, 35 mmol) dropwise such that the temperature remained below -5 °C. After 1.5 hours the mixture was concentrated in vacuo to a volume of 20 ml and treated with pyrrolidine (22 ml, 260 mmol) under reflux for 1.5 hours. The resulting mixture was poured into aqueous sodium carbonate (700 ml) and extracted with dichloromethane (3 x 100 ml) to give, after concentration, an orange oil (15 g) which was distilled to give the N-benzyl diamines (3) as a mixture of three (racemic) isomers (9.3 g, 31 mmol, 97%);
bp 122-155 °C/0.2 mbar; i.r. (neat) 2936, 2791 $cm^{-1}$; m/e ($EI^+$) 303 (5%); 287 (10%), 270 (10%), 84 (100%); δ ($CDCl_3$, $D_2O$, 300 MHz); 7.30 (5H,m); 3.65 (2H,s); 3.75-3.50 (1H,m); 3.36 (s) and 3.32 (s) and 3.29 (s) together are (3H); 2.19 (s) and 2.17 (s) and 2.15 (s) together are (3H); 3.10-1.00 (16H,m); Anal. $C_{19}H_{30}N_2O \cdot 0.33H_2O$ requires C, 74.00; H, 10.02; N, 9.08. Found C, 73.97; H, 9.78; N, 8.82%.

EXAMPLE 4

Diamines (4)

The N-benzyl diamines (3) (3.5 g, 12 mmol) were dissolved in ethanol (50 ml) and treated with 20% palladium hydroxide on carbon (0.94 g) and hydrogen at 50 psi at 60 °C for 2 hours. The mixture was filtered through kieseleguhr and distilled to give the diamines (4) as a mixture of three (racemic) isomers (1.4 g, 6.6 mmol, 55%); bp 84-85 °C/0.3 mbar; i.r. (neat) 3402, 2942 $cm^{-1}$; m/e ($EI^+$) 197 (5%), 180 (10%), 84 (100%); ($CDCl_3$, $D_2O$, 300 MHz), 3.36 (s) and 3.31 (s) and 3.28 (s) together are (3H); 2.38 (s) and 2.37 (s) together are (3H); 3.7-3.2 (1H,m); 2.8-0.9 (17H,m). An analytically pure sample was obtained by treating (4) (212 mg, 1.0 mmol) with p-toluene sulphonic acid (190 mg, 1.0 mmol) in propan-2-ol (1 ml) to give a white solid which was recrystallized from propan-2-ol/diethyl ether to give the mono-p-toluenesulphonate salt (200 mg, 0.50 mmol, 50%), mp 120-136 °C. Anal. $C_{12}H_{24}N_2O \cdot C_7H_8SO_3$ requires C, 59.35; H, 8.39; N, 7.29; S, 8.34. Found C, 58.95; H, 8.24; N, 6.99; S, 8.42%.

EXAMPLE 8

(1α,2α,3α)-2-methoxy-7-oxobicyclo[4.1.0]heptane (1) and (1α, 2β, 6α) isomer (2)

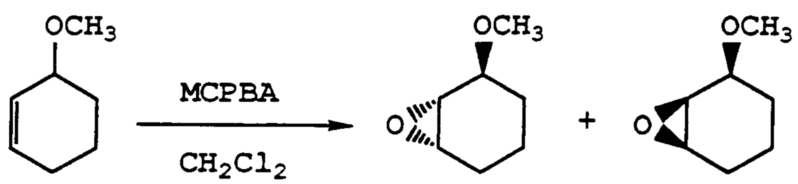


1 2

A solution of 3-methoxycyclohexene (258.11 g, 2.3 mol, Aldrich) in 9.4 L of methylene chloride was cooled in an ice/acetone bath. Solid m-chloroperbenzoic acid (50%, 884.6 g, 2.56 mol) was added in small portions over 30 minutes, keeping the temperature below 3 °C. The reaction was allowed to warm to room temperature overnight. The reaction was then cooled to 15 °, and 4 L of 5% sodium bisulfite was added slowly. After 15 minutes, 205 g of NaOH in 2 L of water was added, and the mixture was stirred for 20 minutes. The aqueous layer was removed, 5 L of saturated $NaHCO_3$ was added, and the mixture was stirred for 20 minutes. The layers were separated and the organic layer was dried over $K_2CO_3$. The solution was filtered and the solvent removed under vacuum. The remaining oil was distilled (80 °-90 °C, 25 mm Hg) to yield 242.8 g (82%) of a 2:1 mixture of 1 and 2. The isomers were separated by vacuum distillation through a 4-ft fractionating column to give a 76% combined overall yield of material with >95% isomeric purity. *Anti*-isomer (major isomer) - $^1H$-NMR ($CDCl_3$) δ 3.5-3.4 (m, 1H), 3.45 (s, 3H), 3.2 (br s, 1H), 3.1 (d, J = 3.7 Hz, 1H), 2.1-2.0 (m, 1H), 1.9-1.7 (m, 2H), 1.5-1.3 (m, 1H), 1.4-1.1 (m, 2H). FTIR (neat): $cm^{-1}$, 2943,

1460, 1101, 933, 802. *Syn*-isomer - $^1$H-NMR ($CDCl_3$): $\delta$ 3.7-3.6 (m, 1H), 3.46 (s, 3H), 3.4-3.2 (m, 2H), 1.9-1.8 (m, 2H), 1.7-1.1 (m, 4H). FTIR - 1459, 1113, 931, 864 $cm^{-1}$. m/e (CI + $CH_4$) 129 (23%), 111 (99%), 97 (100%), 85 (25%). The structure of the antiisomer was confirmed by COSY and NOESY experiments.

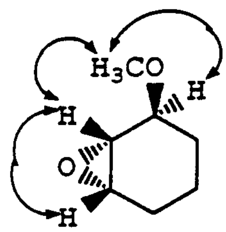

NOE Observed

EXAMPLE 9

(1$\alpha$,2$\beta$,6$\beta$)-2-methoxy-6-(1-pyrrolidinyl)-cyclohexanol (3)

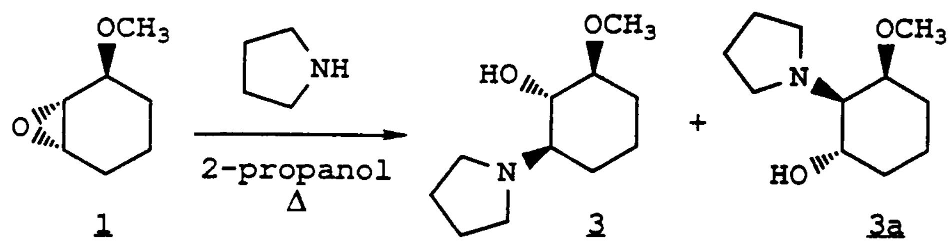


1 3 3a

(major)

Oxirane 1 (20.0 g, 0.156 mol) and pyrrolidine (20.1 g, 0.283 mol) were refluxed in 100 mL of 2-propanol for 6.5 hours. The solvent was removed under vacuum, and the resulting oil was distilled (112-116 °C, 1.1 mm Hg) to give 3 (28.85 g, 0.145 mol, 93%) as a yellow oil. GC showed a 95:5 ratio of isomers. $^1$H-NMR ($d_6$-DMSO): $\delta$ 4.15 (br s, 1H), 3.32 (s, 3H), 3.2-3.1 (m, 1H), 2.96-2.8 (m, 1H), 2.7-2.65 (m, 4H), 2.5-2.3 (m, 1H), 2.0-1.8 (m, 1H), 1.7-1.5 (m, 5H), 1.3-0.9 (m, 3H). FTIR (neat): $cm^{-1}$, ~3300 (br), 1462, 1353, 1298, 1101, 1071, 883. m/e (EI+) 201 (41%), 199 (42%), 184 (61%), 168 (100%), 110 (99%), 70 (62%). Analysis requires: C, 66.29%; H, 10.62%; N, 7.03%. Found: C, 65.96%; H, 10.68%; N, 6.85%. Structure of 3 was verified by COSY and NOESY experiments.

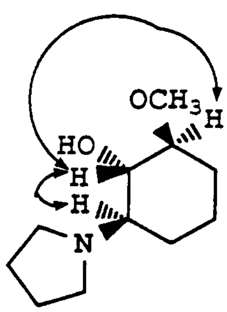

COUPLING OBSERVED

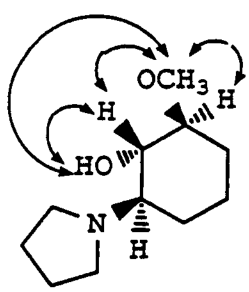

NOE OBSERVED

EXAMPLE 10

(1α,2β,3β)-N-[3-methoxy-2-(1-pyrrolidinyl)-cyclohexyl]-N-methylbenzenemethanamine (4)

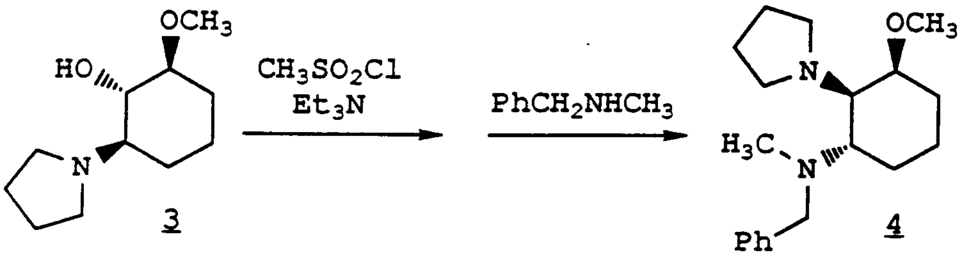


The aminoalcohol 3 (27.47 g, 0.138 mol) was dissolved in 270 mL $CH_2Cl_2$ and 23 mL triethylamine. The solution was cooled to 0°, and 10 mL methanesulfonyl chloride was added in one portion. The temperature rose rapidly to 28°. when the temperature returned to 20°, 2.6 mL more methanesulfonyl chloride was added dropwise with cooling. A solid precipitate formed, and 50 mL more $CH_2Cl_2$ was added to facilitate stirring. After 4 hours, the reaction was complete by GC. The reaction mixture was poured into 450 mL $CH_2Cl_2$ and washed three times with 450 mL water. The organic layer was dried over magnesium sulfate, in the cold, overnight. The solution was filtered and the solvent removed under vacuum, to give 28.68 g of a solid. This solid was dissolved in 64 mL of N-benzylmethylamine and heated to 93°C for 4 hours. The reaction mixture was poured into 100 mL 10% $K_2CO_3$ and extracted three times with $CH_2Cl_2$. The organic layer was dried over $K_2CO_3$, filtered, and the solvent removed under vacuum. The product was vacuum distilled to give a yellow oil, 4 (28.83 g, 0.095 mol, 69%) bp 140-160°/2.8 mm Hg. $^1$H-NMR ($CDCl_3$): δ 7.5-7.1 (m, 5H), 4.0-3.8 (br m, 1H), 3.63 (s, 2H), 3.30 (s, 3H), 3.15 (td, J=3.7, 11 Hz, 1H), 3.1-2.7 (br m, 5H), 2.07 (s, 3H), 2.05-2.85 (m, 2H), 2.85-2.65 (br, 4H), 1.6-1.4 (m, 2H), 1.4-1.0 (m, 2H). Analysis requires: C, 75.45%; H, 10.00%; N, 9.26%. Found: C, 75.30%; H, 9.98%; N, 8.99%.

EXAMPLE 11

(1α,2β,3β)-N-[3-methoxy-2-(1-pyrrolidinyl)cyclohexyl]-N-methylbenzene methanamine monohydrochloride

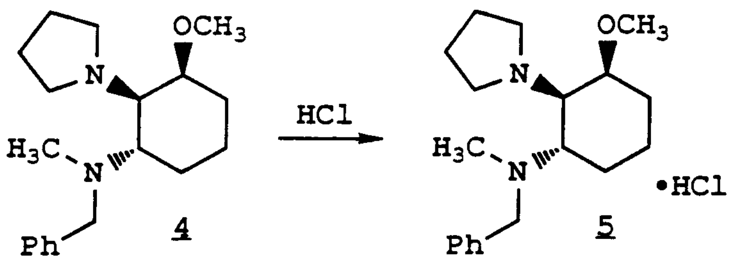


The diamine 4 (21.96 g, 0.0726 mol) was dissolved in 210 mL anhydrous ether and 28 mL of 2.6 M HCl (in 2-propanol) was added dropwise. After addition was complete, the reaction was stirred 10 minutes at room temperature, strongly cooled (dry ice acetone bath), filtered, and the filter cake washed with cold anhydrous ether. The monohydrochloride salt (16.40 g, 0.0484 mol) was obtained as a white powder after drying. The mother liquor was washed with 15% NaOH, dried over $MgSO_4$, filtered, and the solvent removed under vacuum, to give 6.71 g of 4. The yield of mono HCl salt (5) was 96% after recovery of 4. The crude salt was used for the next step. $^1$H-NMR ($d_6$-DMSO): δ 10.0 (br, 1H), 7.4-7.2 (m, 5H), 4.14 (s,1H), 3.8-3.5 (m, 3H), 3.4-3.1 (m, 8H; includes s, 3H at 3.27, and br at 3.4), 2.03 (s+br, 4H), 1.9( br, m, 4H), 1.6-1.0 (m, 4H). FTIR (KBr): $cm^{-1}$, 3454, 2938, 2603, 1476, 1087, 1020, 752, 706. m/e (EI+) 303 (14%), 302 (23%), 287 (42%), 160 (32%), 134 (33%), 123 (76%), 110 (35%), 91 (71%), 84 (100%). Analysis requires: C, 67.33%; H, 9.22%; N, 8.27%; Cl, 10.46%. Found: C, 66.90%; H, 9.18%; N, 7.98%; Cl, 10.65%; $H_2O$, 0.17%. GC showed that this material was 94% pure.

EXAMPLE 12

(1α,2β,3β)-3-methoxy-N-methyl-2-(1-pyrrolidinyl)-cyclohexanamine

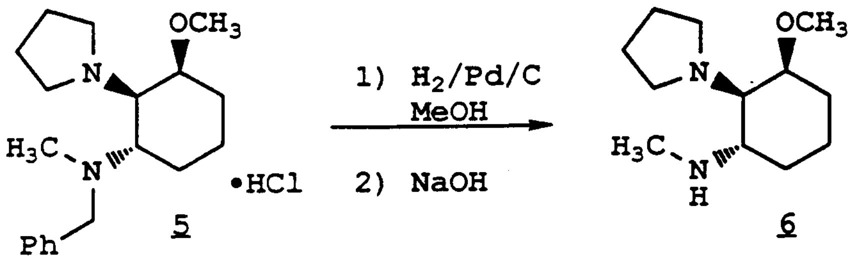


The crude salt 5 (16.40 g, 0.0487 mol) was reduced with 1.5 g 20% Pd/C in 500 mL MeOH. The catalyst was filtered off and the solvent removed under vacuum. Dilute NaOH and ether were added to the resulting solid, and the layers were separated. The aqueous base was washed twice with ether, and the combined ether washings were dried over $Na_2SO_4$. The solution was filtered and the solvent removed under vacuum, to give the diamine 6 (9.45 g, 0.0445 mol, 91% yield). The crude amine was taken on to the next step, since by GC the material was 97% pure. A portion of the material was purified by Kulgelrohr distillation (100 °C, 1.2 mm Hg). $^1$H-NMR ($CDCl_3$): δ 3.8 (m, 1H), 3.24 (s, 3H), 2.8-2.6 (m, 5H), 2.6-2.45 (m, 2H), 2.40 (s, 3H), 2.2-1.9 (m, 2H), 1.8-1.4 (m, 6H), 1.3-0.9 (m, 2H). FTIR (neat): $cm^{-1}$, 3319 (br), 2932 (s), 1459, 1437, 1360, 1095 (s). m/e (EI+) 212 (2.5%), 197 (8.6%), 180 (6.1%), 123 (100%), 108 (22%), 84 (65%). Analysis requires: C, 67.88%, H, 11.39%; N, 13.20%. Found: C, 68.17%; H, 11.84%; N, 13.27%. GC showed the product to be 94% pure.

EXAMPLE 13

(1α,2β,3β)-N-[3-methoxy-2-(1-pyrrolidinyl)-cyclohexyl]-N-methyl-4-benzofuranacetamide monohydrochloride

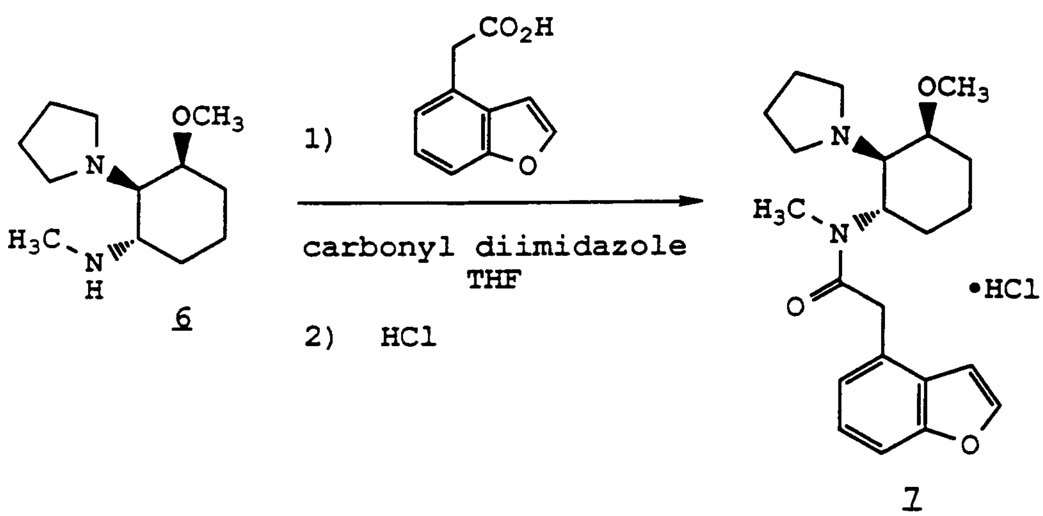


Benzofuranacetic acid (1.95 g, 0.0111 mol) was dissolved in 35 mL THF. Carbonyldiimidazole (1.81 g, 0.0112 mol) was added in one portion and the reaction was stirred under nitrogen for 2.5 hours. The amine 6 (2.13 g, 0.0100 mol) in 10 mL THF was then added and the solution was stirred overnight. The solvent was removed under vacuum and 80 mL $CH_2Cl_2$ was added. This solution was washed with 30 mL $H_2O$, twice with 30 mL 5% NaOH, and finally with 30 mL $H_2O$. The solution was dried over $MgSO_4$, filtered, and the solvent removed under vacuum. The resulting viscous yellow oil gradually solidified to give 2.22 g, (54% yield). $^1$H-NMR ($CDCl_3$): δ 7.6 (d, J = 2.2 Hz, 1H), 7.4-7.36 (d, J = 8.1 Hz, 1H), 7.25-7.1 (m, 2H), 7.0-6.9 (dd, J = 2.7 Hz, 15.5 Hz, 1H), 4.4-4.2 (m, 1H), 4.18-4.1 (d, J = 15 Hz, 1H), 4.0-3.8 (m, 2H), 3.28 + 3.21 (two

singlets, 3H), 2.81 + 2.76 (two singlets, 3H), 2.76-2.5 (m, 8H), 2.1-1.9 (m, 1H), 1.9-0.9 (m, 9H). FTIR (KBr): $cm^{-1}$, ~3500 ($H_2O$), 2936, 1638 (vs), 1434, 1138, 1100, 761 (s). m/e (CI+, $CH_4$) 399 (12%), 371 (100%), 338 (5%), 213 (9%), 181 (7%). Analysis requires: C, 71.32%; H, 8.16%; N, 7.56%. Found: C, 70.99%; H, 8.40%; N, 7.87%. This material was dissolved in isopropanol and 3 mL of 7.3 M HCl in isopropanol was added. Dry ether was added until the solution became cloudy. This material was allowed to stand at room temperature for 3 hours and placed in a freezer overnight. The resulting solid was filtered, washed with dry ether, and dried under vacuum at 50 °C. The solid was recrystallized in isopropanol and then lyophilized from water to give 0.80 g of 7. Analysis requires: C, 64.93%; H, 7.68%; N, 6.89%; Cl, 8.71%. Found: C, 63.32%; H, 7.70%; N, 6.53%; Cl, 8.59%; $H_2O$, 3.85%. $^1$H-NMR ($d_6$-DMSO): δ 10.35 (br, 1H), 7.93-7.91 (d, J=2 Hz, 1H), 7.47-7.42 (d, J=8.2 Hz, 1H), 7.27-7.18 (m, 2H), 7.1-7.0 (d, J=7.3 Hz, 1H), 4.9-4.7 (br, 1H), 4.27-4.2 (d, J=16 Hz, 1H), 4.96-3.88 (d, J=16 Hz, 1H), 3.7-3.3 (m, 4H), 3.3-3.1 (m and s at 3.19, 4H), 3.1-2.9 (m and s at 3.00, 4H), 2.1-1.3 (m, 10H).

EXAMPLE 14

(1α,2β,6β)-2-methoxy-6-(1-pyrrolidinyl)cyclohexanol or (1α,2β,3α)-3-methoxyl-2-(1-pyrrolidinyl) cyclohexanol

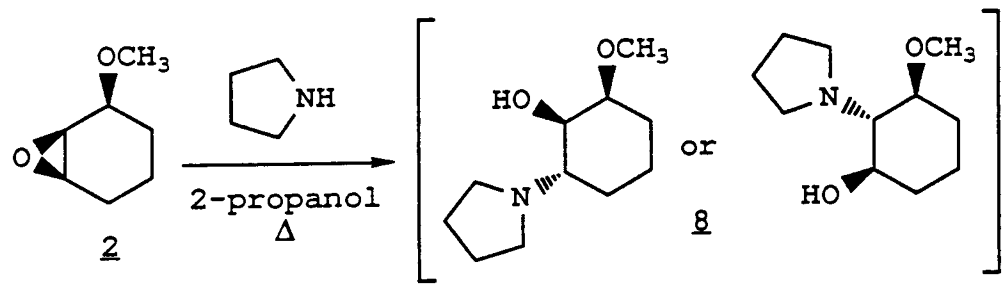


2

or

8

The oxirane 2 (16.78 g, 0.131 mol) was dissolved in 178 mL 2-propanol, and pyrrolidine (11.94 g, 0.168 mol) was added. The solution was refluxed under $N_2$ for 20 hours. The solvent was removed under vacuum. Only one product was seen by GC. The yellow oil was kugelrohr distilled (80-120 °C, 1.2 mm Hg) to yield 8 (23.61 g, 0.118 mol, 90%). $^1$H-NMR ($CDCl_3$): δ 3.8-3.7 (m, 1H), 3.7 (br, 1H), 3.6-3.45 (m, 1H), 3.41 (s, 3H), 3.0-2.8 (td, J=3.5, 10 Hz, 1H), 2.7-2.5 (m, 4H), 2.0-1.85 (m, 1H), 1.85-1.1 (m, 8H). m/e (EI+) 200 (77%), 199 (27%), 184 (38%), 168 (54%), 110 (100%), 70 (76%).

EXAMPLE 15

(1α,2β,3α)-N-[3-methoxy-2-(1-pyrrolidinyl)cyclohexyl]-N-methylbenzenemethanamine

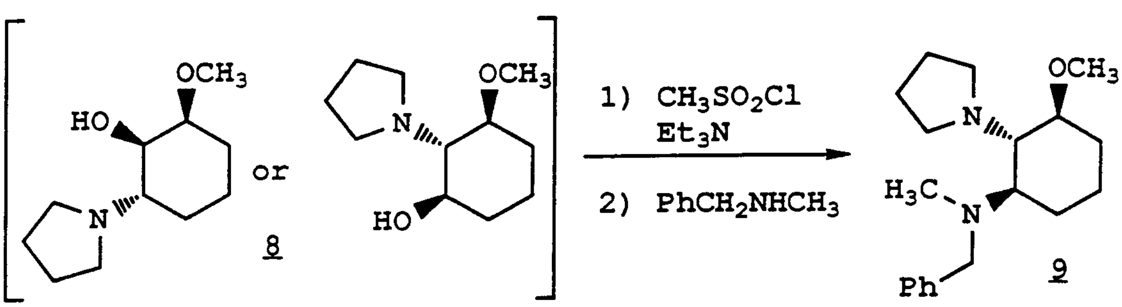


or

8

9

The alcohol 8 (23.38 g, 0.120 mol) was dissolved in $CH_2Cl_2$ and triethylamine (20 mL) was added. The solution was cooled to -5 °C under $N_2$, and methanesulfonyl chloride was added dropwise, keeping the temperature between 0 °C and 5 °C. The reaction was allowed to warm to room temperature overnight. The reaction mixture was then poured into 700 mL $CH_2Cl_2$ and washed with 350 mL $H_2O$, and then 700 mL $H_2O$. The organic layer was dried over $MgSO_4$, filtered, and the solvent removed under vacuum. N-benzylmethylamine (55 mL) was added, and the mixture was heated to 95 °C (under $N_2$) for 20 hours. The

reaction mixture was poured into 300 mL 10% $K_2CO_3$. The product was extracted into $CH_2Cl_2$, dried over $MgSO_4$, filtered, and the solvent removed under vacuum. The yellow oil was kugelrohr distilled, collecting the fraction boiling from 110-160 °C (1.0 mm Hg). The diamine 9 (25.80 g, 0.0853 mol, 71% yield) was obtained as a viscous yellow oil. $^1$H-NMR ($CDCl_3$): $\delta$ 7.5-7.1 (m, 5H), 3.73 (s, 2H), 3.36 (s, 3H), 3.3-3.1 (m, 1H), 3.0-2.7 (m, 4H), 2.6-2.5 (m, 1H), 2.22 (s, 3H), 2.2-2.0 (m, 1H), 1.9-1.0 (m, 9H).

EXAMPLE 16

(1α,2β,3α)-3-methoxy-N-methyl-2-(1-pyrrolidinyl)-cyclohexanamine

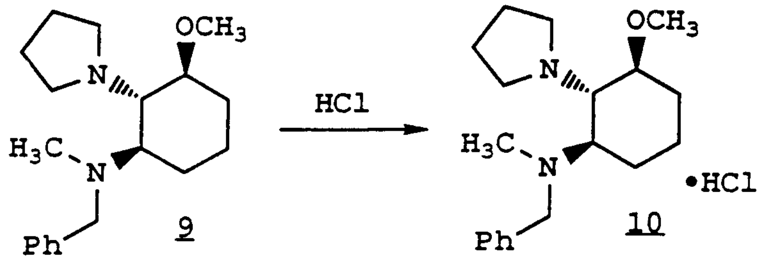

9
10

The crystalline diamine 9 (3.75 g, 12.4 mmol) was dissolved in 75 mL of methanol with 0.5 g 20% Pd/C, and reduced with 50 psi of $H_2$ for 18 minutes at ambient temperature. The crude product showed a single peak by GC. The catalyst was filtered off and the solvent removed under vacuum to give 11 (2.62 g, 12.3 mmol, 99% yield). $^1$H-NMR ($CDCl_3$): $\delta$ 3.45-3.35 (m, 1H), 3.31 (s, 3H), 3.0-2.8 (m, 2H), 2.8-2.6 (m, 2H), 2.6-2.4 (m, 2H), 2.38 (s, 3H), 2.3-1.9 (m, 3H), 1.9-1.6 (m, 5H), 1.3-0.9 (m, 3H). FTIR (neat): cm$^{-1}$, 3304, 2935, 1440, 1105 (s), 850. m/e (EI+) 123 (67%), 110 (16%), 108 (17%), 84 (100%), 71 (11%), 70 (25%), 55 (15%), 42 (39%). The structure of 11 was verified by decoupling, COSY, and NOESY experiments. Alternatively the monohydrochloride salt 10 (19.73 g, 0.0586 mol), was dissolved in 500 mL methanol, with 1.5 g 20% Pd/C, and reduced at 50 psi $H_2$ for 19 hours at ambient temperature. The reaction mixture was filtered, and the solvent removed under vacuum. Aqueous base (250 mL 15% NaOH) was added to the resulting solid, and the mixture was extracted twice with 150 mL $CH_2Cl_2$ and once with 200 mL ether. The combined organic layers were dried over $MgSO_4$, filtered, and the solvent was removed under vacuum. The product was kugelrohr distilled collecting the fraction 80°-120°C (0.1 mm Hg) to give 11 (8.38 g, 0.0395 mol, 67% yield) spectrally identical to material resulting from debenzylation of the free base.

EXAMPLE 17

(1α,2β,3α)-N-[3-methoxy-2-(1-pyrrolidinyl)cyclohexyl]-N-methylbenzenemethanamine monohydrochloride

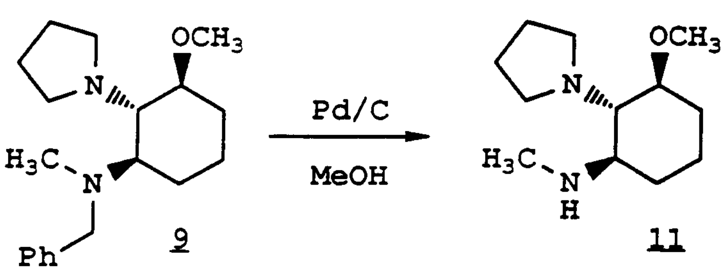

9
11

The diamine free base 9 (25.11 g, 0.0830 mol) in 250 mL of anhydrous ether, was treated with 11.2 mL of HCl in isopropanol (0.267 g HCl/mL). The resulting monohydrochloride salt, 10, a hygroscopic white powder, was collected by filtration. A second crop of 10 was obtained by washing the mother liquor with 15% NaOH, drying over $MgSO_4$, evaporating to an oil, and similar treatment with HCl. The combined yield of 10 was 19.78 g. Compound 10 was hygroscopic.

The mother liquor from the second crop of 10 was washed with 15% NaOH, dried, and evaporated as before, to give a yellow oil. On standing for three days, the free base, 9 (3.81 g), crystallized from this oil, mp 59°-60°C. 9: $^{1}$H-NMR ($CDCl_3$): δ 7.4-7.1 (m, 5H), 3.72 (s, 2H), 3.35 (s, 3H), 3.3-3.0 (m, 1H), 3.0-2.7 (m, 5H), 2.6-2.5 (td, J=3.6, 11.5 Hz, 1H), 2.22 (s, 3H), 2.2-2.0 (m, 1H), 1.9-1.7 (m, 6H), 1.5-1.0 (m, 3H). $^{13}$C-NMR (ppm) 141.2, 128.5, 128.0, 126.4, 81.3, 63.8, 62.8, 59.4, 56.2, 47.9, 36.7, 30.6, 27.8, 24.1, 21.7. FTIR (KBr): $cm^{-1}$, 1603, 1496, 1042, 956, 903, 833, 747. m/e (EI+) 303 (M+1, 33%), 302 (M, 39%), 287 (31%), 160 (33%), 123 (56%), 91 (94%), 84 (100%). Both the free base 9 and the monohydrochloride 10 were successfully used in the next step.

EXAMPLE 18

(1α,2β,3α)-N-[3-methoxy-2-(1-pyrrolidinyl)-cyclohexyl]-N-methyl-4-benzofuranacetamide monohydrochloride

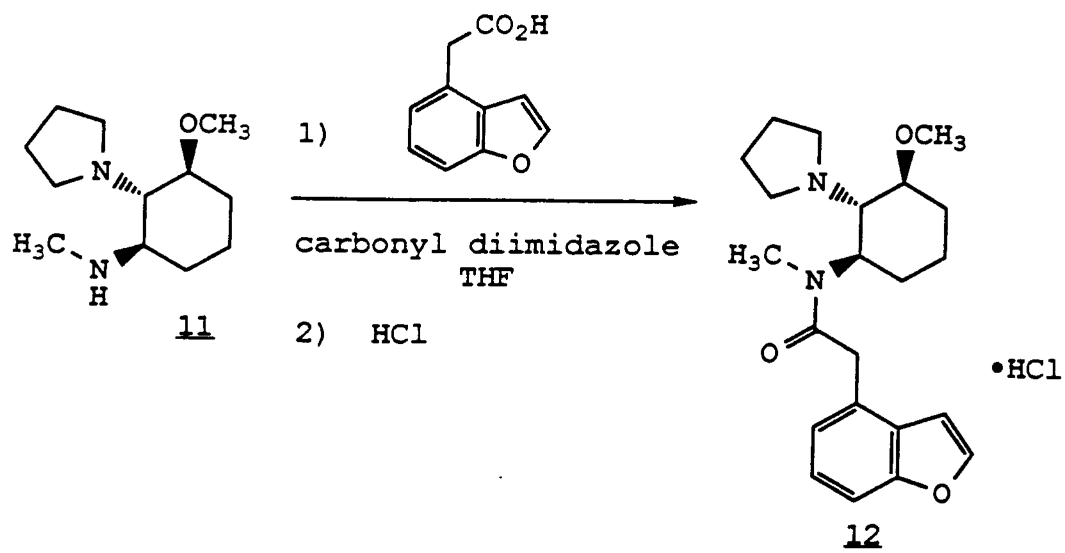


Benzofuranacetic acid (1.51 g, 8.57 mmol) was dissolved in 20 mL anhydrous THF (under $N_2$), and carbonyldiimidazole (1.39 g, 8.57 mmol) was added in one portion. Gas evolution was observed. The reaction was stirred overnight at ambient temperature. The amine 11 (1.40 g, 6.59 mmol) in 10 mL THF was then added, and the reaction was stirred for 6 hours. The reaction mixture was poured into $CH_2Cl_2$, and washed once with water, twice with 15% NaOH, and a final time with water. The combined aqueous layers were extracted twice with ether. All organic layers were combined and dried over $MgSO_4$, filtered, and the solvents removed under vacuum. A white solid (2.22 g, 6.0 mmol, 90% yield) mp 97-101°C was obtained. $^{1}$H-NMR ($CDCl_3$): δ 7.63 (s, 1H), 7.4 (d, J=8 Hz, 1H), 7.35-7.0 (m, 2H), 6.9-6.8 (m, 1H), 4.7-4.5 (br m, 1H), 4.1-3.8 (m, 2H), 3.6-3.1 (m, 4H; includes s at δ 2.8), 2.4-2.2 (m, 1H), 1.8-0.8 (m, 9H). FTIR (KBr): $cm^{-1}$, 3500 (br), 2854, 1636, 1533 (w), 1431, 1250, 1086, 765 (water was evident in the IR). m/e (EI+) 371 (1%), 355 (6%), 338 (5%), 181 (37%), 149 (89%), 131 (28%), 110 (21%), 84 (100%), 70 (29%). Analysis requires: C, 71.32%; H, 8.16%; N, 7.56%. Found: C, 70.58%; H, 8.18%; N, 7.83%. HPLC (210 nm) showed that the compound was 99.3% pure. This material (2.13 g, 5.7 mmol) was dissolved in 50 mL ether and 10 mL acetonitrile. Then 2.43 g of 16% HCl/ether solution was added dropwise. A precipitate gradually formed. The solution was cooled on ice and filtered. The solid was recrystallized in isopropanol/ether, and lyophilized in water to give the hydrochloride salt as a glassy solid (1.4 g, mp. 60°-70°C, 97.3% pure by GC). $^{1}$H-NMR ($d_6$-DMSO): δ 9.4 (br, 1H), 7.95-7.9 (d, J=2.2 Hz, 1H), 7.5-7.4 (d, J=8 Hz, 1H), 7.3-7.1 (m, 2H), 7.1-7.0 (d, J=7.3 Hz, 1H), 4.6 (br, 1H), 4.3-4.1 (d, J=16 Hz, 1H), 4.0-3.9 (d, J=16 Hz, 1H), 3.9-3.6 (m, 4H), 3.5-3.1 (m, includes s at 3.40 (water), and s at 3.31), 3.0 (s, 3H), 2.3-2.1 (m, 1H), 2.05-1.1 (m, 9H). Analysis requires: (corrected for 5.5% water) C, 61.35%; H, 7.87%; N, 6.50%; Cl, 8.23%. Found: C, 61.2%; H, 8.10%; N, 6.47%; Cl, 8.43%; $H_2O$, 5.46%.

EXAMPLE 19

(1α,2β,6β)-2-methoxy-6-[methyl(phenylmethyl)amino]-cyclohexanol (17)

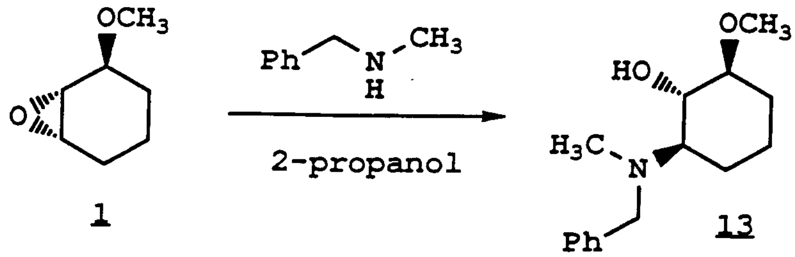


1 13

Oxirane 1 (20.08 g, 0.157 mol) and N-methylbenzylamine (30.24 g, 0.25 mol) were dissolved in 110 mL of 2-propanol and heated to reflux, under $N_2$, for 18 hours. The solvent was removed under vacuum, and the product was Kugelrohr distilled, removing the material that distilled below 100 °C at 1.0 mm Hg, and collecting the fraction boiling between 100 °C-140 °C at 1.0 mm Hg. A colorless oil 13 (35.42 g, 90%) was obtained. The product showed a single peak by GC. $^1$H-NMR ($d_6$-DMSO): δ 7.4-7.1 (m, 5H), 4.17 (s, 1H), 3.74-3.68 (d, J = 13.4 Hz, 1H), 3.55-3.45 (d, J = 13.4 Hz, 1H), 3.33 (s, 3H), 3.3-3.2 (m, 1H), 3.0-2.85 (m, 1H), 2.4-2.3 (m, 1H), 2.13 (s, 3H); 2.0-1.8 (m, 1H), 2.8-2.6 (m, 2H), 1.4-0.9 (m, 3H). $^{13}$C-NMR ($d_6$-DMSO): δ 140.1, 128.7, 128.4, 126.9, 83.7, 73.9, 65.9, 58.1, 57.1, 36.6, 29.4, 22.9, 21.4. FTIR (neat): $cm^{-1}$, 3400 (br), 2938, 2864, 1496, 1454, 1121, 1096, 881, 743, 701. m/e (EI+) 249 (m, 17%), 234 (20%), 218 (22%), 160 (22%), 120 (5%), 91 (100%), 65 (7%), 42 (13%). Analysis requires: C, 72.25%; H, 9.30%; N, 5.62%. Found: C, 72.23%; H, 9.22%; N, 5.47%. Structure was verified by COSY, HETCOR, LRHETCOR, and DEPT NMR techniques.

EXAMPLE 20

(1α,2α,6β)-N-[2-methoxy-6-(1-pyrrolidinyl)cyclohexyl]-N-methylbenzenemethanamine

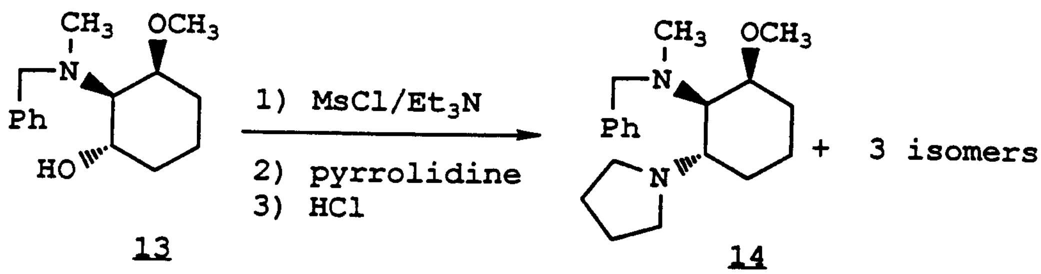


13 14

Amino alcohol 13 (35.08 g, 0.141 mol) was added to 460 mL $CH_2Cl_2$ and 32 mL triethylamine under $N_2$. The resulting solution was cooled to -10 °C. Methanesulfonylchloride (13 mL) was added dropwise, maintaining a temperature below -5 °C. After the addition was complete, the cold bath was removed, and the reaction stirred for 5 hours, at which time all the starting material was consumed, as shown by GC. The reaction mixture was poured into a separatory funnel with 500 mL $CH_2Cl_2$. The organic layer was washed three times with 250 mL $H_2O$, dried over $MgSO_4$, filtered, and the total volume of the solution reduced to approximately 250 mL under vacuum. To this solution, 97 mL of pyrrolidine was added, and allowed to stand at room temperature for 16 hours, then heated to reflux for 1.5 hours. The reaction mixture was poured into 800 mL of 10% $Na_2CO_3$ and extracted with 500 mL $CH_2Cl_2$, then 400 mL $CH_2Cl_2$, then 500 mL $CH_2Cl_2$. The combined organic layers were dried over $MgSO_4$, filtered, and the solvent removed under vacuum. The crude oil was Kugelrohr distilled to remove colored impurities to give 18.21 g (43%) of a pale yellow oil. A GC on the distillate showed a mixture of four products in a ratio of 1:4:7:4 in order of increasing retention time.

This crude oil was dissolved in 95 mL ether and treated with 3 mL of 2.6 N HCl in isopropanol. The solution was decanted away from the brown solids that formed and additional 50 mL of ether was added to the solution and 6 mL more of the HCl/isopropanol solution was added dropwise, and the resulting solution placed in a freezer for 3 weeks. The crystals that formed were filtered, and several crystal forms were observed. The largest brown color crystals were mechanically separated. These were treated with dilute NaOH and extracted with ether. The organic layer was dried over $Na_2SO_4$, filtered, and the solvent removed under vacuum. The oil obtained was purified by flash chromatography ($SiO_2$) using 1.5% MeOH/$CHCl_3$ and saturated with $NH_4OH$) to yield 14 (0.45 g, 0.0015 mol). $^1$H-NMR ($CDCl_3$): δ 7.5-7.1 (m, 5H); 4.0-3.8 (d, J = 13.5 Hz, 1H); 3.86-3.80 (m, 1H); 3.77-3.72 (d, J = 13.5 Hz, 1H); 3.3 (s, 3H); 3.3-3.2 (td, J = 3.3, 11.3 Hz, 1H); 2.8-2.6 (m, 4H); 2.6-2.5 (dd, J = 2.3, 10.8 Hz); 2.37 (s, 3H); 2.0-1.8 (m, 2H); 1.8-1.6 (m, 4H); 1.6-1.4 (m, 2H); 1.4-1.0 (m, 2H). $^{13}$C-NMR ($d_6$-DMSO): 141.9, 128.2, 127.8, 126.2, 79.0, 66.2, 58.0, 54.2, 46.2, 27.4, 23.4, 22.9, 19.0. m/e (EI+) 302 (11.4%), 287 (7.9%), 211 (35.5%), 173 (28.1%), 160 (12.2%), 150 (15.5%), 134 (64.5%), 110 (36.0%), 91 (100%), 84 (23.3%), 70 (21.4%), 44 (30.9%). Analysis requires: C, 75.45%; H, 10.00%; N, 9.26%. Found: C, 75.40%; H, 9.97%; N, 9.17%. Stereochemical and regiochemical assignments were determined by COSY, NOESY, and HETCOR NMR experiments.

EXAMPLE 21

(1α,2α,6β)-2-methoxy-N-methyl-6-(1-pyrrolidinyl)-cyclohexanamine

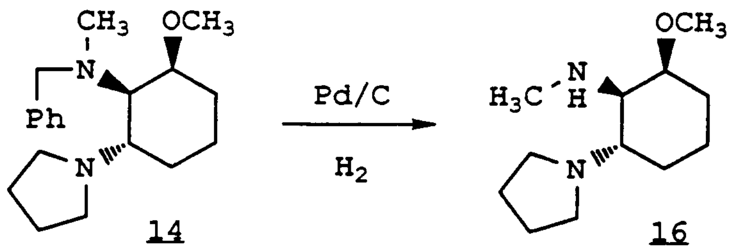


The N-benzyldiamine, 14 (0.45 g, 1.49 mmol) was treated with 0.05 g 20% Pd/C in 75 mL methanol and 50 psi hydrogen at room temperature. The product was filtered and the solvent removed under vacuum. The crude oil was chromatographed (2% MeOH/2% conc. $NH_4OH$/96% $CHCl_3$) to yield 15 (0.23 g, 73% yield). $^1$H-NMR ($CDCl_3$): δ 3.7-3.6 (m, 1H), 3.35 (s, 3H), 2.76-2.6 (m, 1H), 2.6-2.5 (m, 5H), 2.42 (s, 3H), 2.4-2.2 (br, 1H), 1.9-1.3 (m, 10H). m/e (EI+) 212 (8.1%), 181 (22%), 150 (26%), 112 (23%), 111 (24%), 110 (100%), 97 (21%), 96 (21%), 84 (63%), 70 (50%), 55 (22%), 44 (33%), 42 (33%).

EXAMPLE 22

(1α,2α,6β)-N-[2-methoxy-6-(1-pyrrolidinyl)cyclohexyl]-N-methyl-4-benzofuranacetamide monohydrochloride

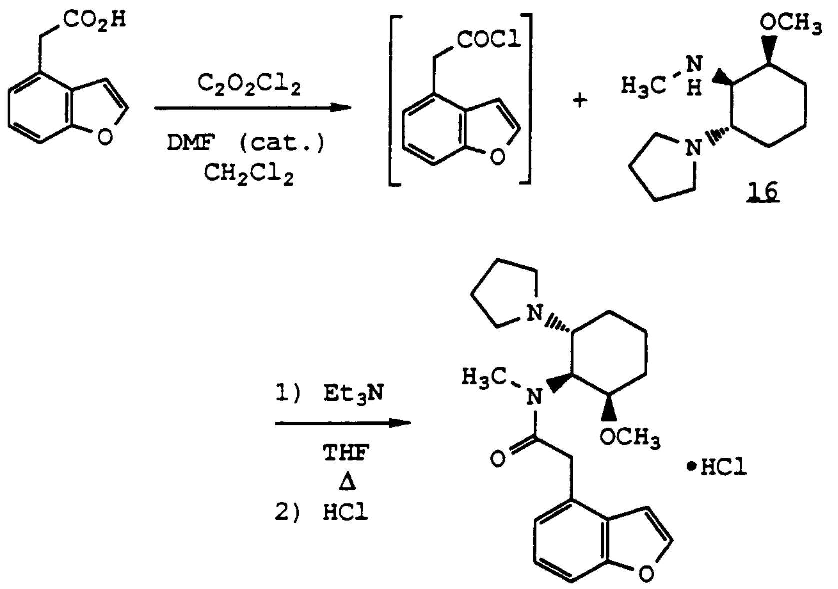


16

17

4-Benzofuranacetic acid (1.01 g, 5.73 mmol) was placed in a 100 mL boiling flask, and dissolved in 50 mL $CH_2Cl_2$ under $N_2$. Oxalyl chloride (1.6 mL) and dimethyl formamide (0.04 g) were added, and gas evolution was observed. The reaction was stirred at 25 °C for 18 hours, and the solvent removed under vacuum. The resulting oil was Kugelrohr distilled (0.07 mm Hg, 90 °C-100 °C) to yield 1.04 g of a yellow oil. This oil was shown by $^1$H-NMR to be a 1:1 mixture of the acid and the acid chloride. This mixture (0.20 g) was dissolved in 10 mL anhydrous THF, and cooled to 0 °C under $N_2$. Then the diamine 15 (0.11 g, 0.52 mmol, dissolved in 2.5 mL THF) and triethylamine (1.0 mL) were added to the acid chloride solution. Then 4-(dimethylamino)pyridine (0.01 g) was added, and the solution stirred at room temperature for 24 hours. The solvent was removed under vacuum, and the crude oil was chromatographed (3% MeOH/1% conc. $NH_4OH/CHCl_3$) twice to yield 16 (0.11 g, 0.30 mmol), as the free base. $^1$H-NMR ($CDCl_3$): δ 7.6 (d, J = 4.9 Hz, 1H), 7.4-7.38 (d, J = 7.9 Hz, 1H), 7.25-7.20 (t, J = 8 Hz, 1H), 7.11-7.08 (d, J = 7.3 Hz, 1H), 6.95 (br s, 1H), 4.6-4.5 (br, 1H), 4.1-3.9 (m, 2H), 3.6 (br s, 1H), 3.3-3.2 (br, 1H), 3.19 (s, 3H), 3.07 (br s, 3H), 2.7-2.5 (br, 2H), 2.1-1.1 (m, 12H). m/e (CI+) 372 (M+2, 25%), 371 (M+1, 100%), 369 (13%), 300 (24%), 181 (63%), 150 (21%), 110 (9%).

This product was dissolved in anhydrous diethyl ether, and treated with 20% HCl/MeOH to form the monohydrochloride salt. This was filtered, and lyophilized to give 16 (0.03 g, 0.08 mmol). $^1$H-NMR ($d_6$-DMSO): δ 9.6 (br, 1H), 7.94-7.92 (d, J = 2.4 Hz, 1H), 7.5-7.4 (d, J = 8.6 Hz, 1H), 7.3-7.2 (t, J = 8 Hz, 1H), 7.18-7.15 (d, J = 2.4 Hz, 1H), 7.1-7.0 (d, J = 7.3 Hz, 1H), 4.8-4.6 (dd, J = 2.4, 12 Hz, 1H), 4.25-4.1 (d, J = 16.5 Hz, 1H), 4.05-3.8 (m, 2H), 3.6-2.8 (m, includes $H_2O$ peak), 2.2-1.2 (m, 10H). m/e (CI+, 1% $NH_3$ in $CH_4$): 372 (M+2, 23%), 371 (M+1, 100%), 300 (32%), 182 (17%), 181 (81%), 150 (37%), 131 (11%), 110 (22%). FTIR (KBr): $cm^{-1}$, 3432 (br), 2945, 1734, 1647 (s), 1457, 1432, 1246, 1124, 1102, 1045, 1009, 765 (s). HRMS (EI+) $C_{22}H_{30}N_2O_3$ - Requires: 370.2256. Found: 370.2246.

## Claims

**1.** A compound of 2-amino-3 or 6-methoxycyclohexyl amide derivatives of the formula

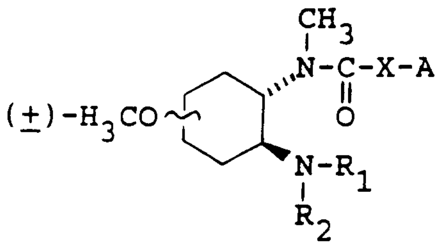

I

wherein the (±)-$H_3CO$~ group is in the 3- or 6- position and the two nitrogen atoms attached to the cyclohexane moiety are trans to one another;
X is $CH_2$ or direct bond,
$R_1$ is methyl,
$R_2$ is selected from the group consisting of:
hydrogen,
alkyl of from one to six carbon atoms,

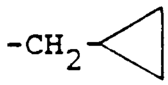

,

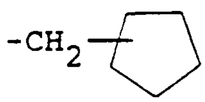

,

-$CH_2CH=CR_3R_4$,
-$CH_2C\equiv R_3$,
-2- or 3-thienyl, or

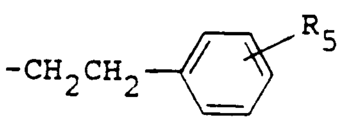

wherein $R_3$ and $R_4$ are each independently hydrogen or methyl,
$R_5$ is selected from the group consisting of:
hydrogen,
fluorine,
chlorine,
bromine,
lower alkyl, and
lower alkoxy;
or $R_1$ and $R_2$ may be taken together with the nitrogen atom to which they are attached to from a ring

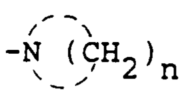

wherein n is an integer of from 3 to 8;
A is selected from the group consisting of:

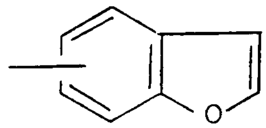
,

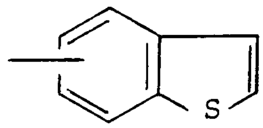
,

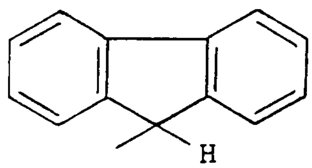
,

phenyl,
phenyl substituted with from one to four halogen atoms,
phenyl substituted with one or two alkyl groups of from one to four carbon atoms,
phenyl substituted with one or two alkoxy groups of from one to four carbon atoms, or
phenyl substituted with one or two alkyl groups of from one to four carbon atoms and one or two halogen atoms;
or a pharmaceutically acceptable acid salt thereof.

**2.** A compound according to claim 1 wherein X is $CH_2$ and A is

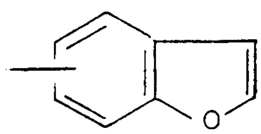
.

**3.** A compound according to claim 2 wherein A is

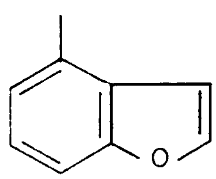
.

**4.** A compound according to claim 1 wherein X is a direct bond and A is

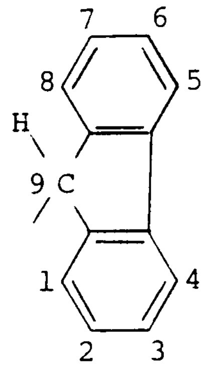

.

5. A compound according to claim 1 selected from the group consisting of (1α,2α,6β)-N-[2-methoxy-6(1-pyrrolidinyl)cyclohexyl]-N-methyl-4-benzofuranacetamide and
(1α,2β,3α)-N-[3-methoxy-2-(1-pyrrolidinyl)-cyclohexyl]-N-methyl-4-benzofuranacetamide
or a pharmaceutically acceptable salt thereof.

6. A compound of claim 5 wherein the salt is a hydrochloride.

7. A method of preparing a compound of claims 1 to 6, wherein A is as defined in claim 1 which comprises reacting a 2-amino-3 or 6-methoxycyclo-hexyl amide compound of formula

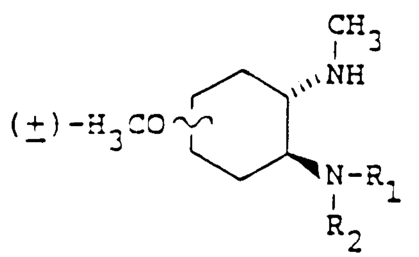

II

wherein $R_1$ and $R_2$ are as defined in claim 1 with A-X-$CH_2$COB wherein B is -Cl, -OH, -$OC_6F_5$ or

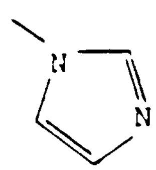

and X is $CH_2$ or a direct bond
and, if desired, converting the product to a pharmaceutically acceptable acid addition salt thereof.

8. A method of preparing a compound of claims 1, 2, 3 or 5 wherein A is

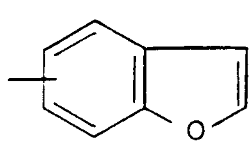

or

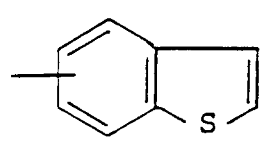

which comprises reacting an amine compound of formula

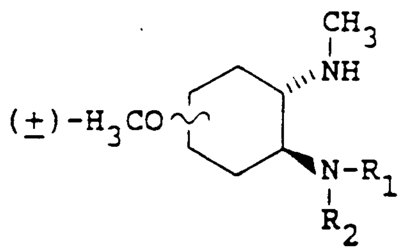

II

wherein $R_1$ and $R_2$ are as defined in claim 1 with A-X-$CH_2$COB wherein B is -Cl, -OH, -$OC_6F_5$ or

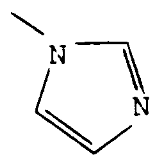

and X is -$CH_2$- or a direct bond
and, if desired, converting the product to a pharmaceutically acceptable acid addition salt thereof.

9. A method of preparing a compound of claims 1, 4 or 6 wherein A is

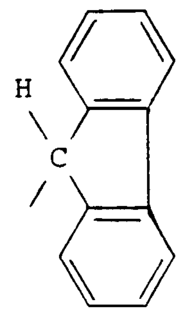

which comprises reacting an amine compound of formula

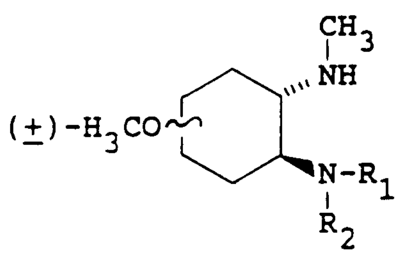

II

wherein $R_1$ and $R_2$ are as defined in claim 1 with

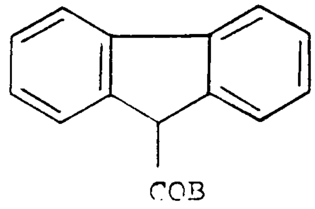

wherein B is -OH, -Cl, or

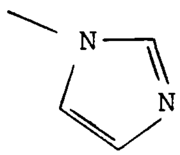

or $OC_6F_5$ and, if desired, converting the product to a pharmaceutically acceptable salt therof.

**10.** A method of preparing a compound according to claim 7, wherein said compound is selected from the group consisting of (1α,2α,6β)-N-[2-methoxy-6-(1-pyrrolidinyl)cyclohexyl]-N-methyl-4-benzofuranacetamide and (1α,2β,3α)-N-[3-methoxy-2-(1-pyrrolidinyl)-cyclohexyl]-N-methyl-4-benzofuranacetamide or a pharmaceutically acceptable salt thereof.

**11.** A method of claim 10, wherein said salt is a hydrochloride.

**12.** A pharmaceutical composition comprising a therapeutically effective amount of a compound as claimed in claims 1 to 6 together with a pharmaceutically acceptable carrier.

**13.** Use of a compound of claims 1 to 6 for the preparation of a pharmaceutical composition for treating stroke or pain in a mammal.

European Patent Office

# EUROPEAN SEARCH REPORT

Application Number

EP 92 10 0432

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | EP-A-0 372 466 (WARNER-LAMBERT COMPANY)<br>* page 2, line 45 - page 4, line 49 * | 1,12 | C07D307/79<br>C07D333/54<br>C07D295/135<br>A61K31/38<br>A61K31/40<br>A61K31/34 |
| Y<br><br>D | EP-A-0 147 085 (WARNER-LAMBERT COMPANY)<br>* page 3, line 10 - page 4, line 28 *<br>& US-A-4 656 182 | 1,12 | |
| Y<br><br>D | EP-A-0 146 297 (WARNER-LAMBERT COMPANY)<br>* page 3, line 10 - page 4, line 12 *<br>& US-A-4 598 087 | 1,12 | |
| Y,D | US-A-4 906 655 (DAVID C.HORWELL ET AL.)<br>* column 1, line 50 - column 4, line 7 * | 1,12 | |
| Y | GB-A-2 096 607 (THE UPJOHN COMPANY)<br>* page 1 - page 3, line 41 * | 1,12 | |
| | ----- | | TECHNICAL FIELDS SEARCHED (Int. Cl.5) |
| | | | C07D |

The present search report has been drawn up for all claims

| Place of search | Date of completion of the search | Examiner |
|---|---|---|
| BERLIN | 09 SEPTEMBER 1992 | KYRIAKAKOU G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)